# EUROPEAN PATENT SPECIFICATION

(11) **EP 0 448 740 B1**
(45) Date of publication and mention of the grant of the patent: **18.10.1995**
(21) Application number: 90105676.2
(22) Date of filing: 26.03.1990
(51) Int. Cl.: C07C 47/02, C07C 33/025, C07C 31/125, A61K 7/46, C11B 9/00

(54) **A perfume composition**
Parfümzusammensetzung
Composition de parfum

(43) Date of publication of application: 02.10.1991
(73) Proprietor: KAO CORPORATION, Chuo-ku Tokyo (JP)
(72) Inventor: Tajima, Katsuhiko, Funabashi-shi, Chiba (JP); Tanaka, Sachio, Ichinomiya-shi, Aichi 491-02 (JP)
(74) Representative: Kraus, Walter, Dr.

(56) References cited:
- FR-A- 361 698
- FR-A- 1 562 796
- FR-A- 2 189 356

## Description

The present invention is directed to a perfume composition comprising a methyl branched aliphatic compound, and a method for imparting a fragrance by using such compounds and compositions.

Up to the present, many aliphatic aldehydes or aliphatic alcohols are known to be useful as perfume materials. For example, with respect to aliphatic aldehydes having 9-11 carbon atoms, the straight chain aldehydes are known to be useful. With respect to aliphatic aldehydes having one branched methyl group, methylhexylacetoaldehyde (2-methyl octanal), methylheptylacetoaldehyde (2-methyl nonanal), methyloctylacetoaldehyde (2-methyl decanal) and methylnonylacetoaldehyde (2-methylundecanal) are known as perfume materials. Furthermore, some aliphatic aldehydes having one branched methyl group at the β-position to aldehyde carbon, have been reported only by D. Hagena, K. Bauer et al. [Fragrance and Flavor Substances, 1980, D&PS, Verlag].

On the other hand, with respect to aliphatic alcohols having 9-11 carbon atoms, there are only terpene alcohols such as linalool, geraniol, terpineol, etc., n-nonanol, n-decanol, n-undecanol, which are known as perfume materials.

FR-A-1 562 796 relates to a perfume composition which comprises a compound of the formula R¹R²CH(CH₂)₂CH(R³)CH₂R⁴ wherein R¹ is C₁-C₄ alkyl, R² is C₁-C₅ or phenyl, R¹ and R² form together a four- or five-membered chain, R³ is hydrogen or methyl or ethyl and R⁴ is CH₂OH or CHO.

It is known that the fragrances of perfume compounds are usually quite differentiated by their differences in functional groups or by their small differences in structure.

Under these circumstances, the present inventors have synthesized many aliphatic alcohols and aldehydes having branched methyl groups and have evaluated their fragrances for the purpose of developing new perfumes and, as a result, found that the aliphatic compound represented by formula (I) and having one methyl group branched at the vicinity at the end of molecule makes the fragrance of various combination perfumes more natural-like when they are used in perfume mixtures. The present invention was accomplished based on the above findings.

Accordingly, the present invention provides for a combination perfume composition which comprises a perfume base; and a fragrance emitting effective amount of a compound selected from the group consisting of 8-methyl nonanal, 8-methyl decanal, 9-methyl decanal, 8-methyl nonanol, 8-methyl decanol, 9-methyl decanol, 8-methyl-6-nonen-1-ol and 9-methyl-6-decen-1-ol.

These compounds have the Formula (I) 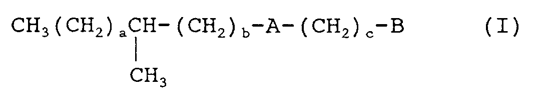
wherein A represents a -CH₂CH₂- or a -CH=CH- group, B represents a -CH₂OH or a -CHO group, b represents 0 or 1, a + c is 4 or 5 and a is 0 or 2.

Methyl branched aliphatic compounds (I), which are useful as a material as a component for perfume compositions, can be prepared according to the process by the following reaction formula: 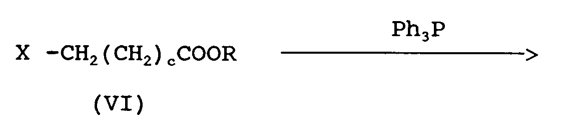 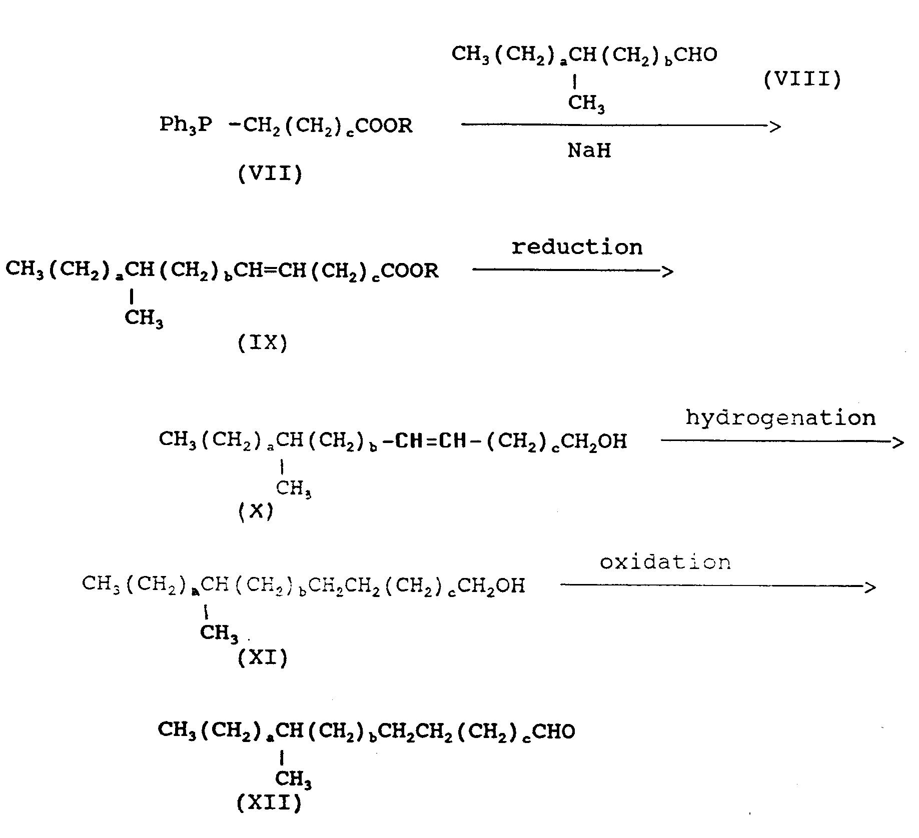
wherein X represents a halogen atom, Ph represents a phenyl group, R represents an alkyl group, and a, b and c have the same meaning as defined above.

Namely, methyl branched unsaturated fatty acid esters (IX) can be prepared by reacting fatty acid esters (VI) having a halogen atom at the end of the molecule with triphenyl phosphine to obtain phosphonium salt (VII), and running the Wittig reaction between the resultant product and the α-methyl branched aldehyde (VIII). By reducing the methyl branched unsaturated fatty acid ester (IX), a methyl branched aliphatic unsaturated alcohol, which is included in the methyl branched aliphatic compound of the formula (I), can be prepared. The methyl branched aliphatic alcohol (XI) can then be obtained by hydrogenating compound (X); furthermore, by oxidizing compound (XI), a methyl branched aliphatic aldehyde (XII) can be prepared.

At first, the preparation of a methyl branched unsaturated fatty acid ester (IX) can be practiced under usual Wittig reaction conditions. Namely, compound (VI) can be reacted with triphenyl phosphine in an inert solvent such as acetonitrile, methyl branched aldehyde is then added, and sodium hydride is then carefully added.

The reduction of methyl branched unsaturated fatty acid ester (IX) can be preferably practiced by using a reducing agent such as aluminum lithium hydride, or sodium borohydride.

Hydrogenation of methyl branched aliphatic unsaturated alcohol (X) can be preferably practiced by using palladium carbon, Raney-nickel, Rubidium carbon, Rhodium carbon, etc., as a catalyst.

Oxidation of methyl branched aliphatic alcohol (XI) can be preferably practiced by using an oxidizing agent such as a pyridinium chlorochromate, chromic acid mixture.

The following is an exemplification of the methyl-branched aliphatic compound used in the perfume composition according to present invention: 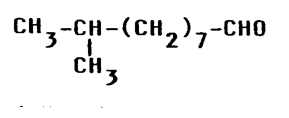
The amount of the methyl branched aliphatic compounds (I) in the perfume composition of the present invention is preferably in the range of 10 ppm to 5 weight %.

Because the methyl branched aliphatic compounds of the formula (I) of the present invention possess citrus or floral odors, as indicated in the test described below, and because their fragrances are extremely strong, they are useful as perfume materials. Accordingly, the perfume compositions of the present invention are applicable in a variety of combination perfumes.

The perfume composition of the present invention can be combined into a variety of base compounds complying with the purpose of the invention.

### [Example]

The present invention is described in detail by way of the following examples and tests.

### Example 1

### Preparation of 8-methyl-6-nonen-1-ol [in formula (I), A= -CH=CH-, B= -CH₂OH, a=b=0, c=4]:

223.12g (1 mol) of ethyl 6-bromohexanoate and 275.4g (1.05 mol) of triphenyl phosphine were refluxed for 36 hours in 1.5 l of acetonitrile. After the reaction was terminated, 500 ml of acetonitrile was distilled off, then the reaction mixture was dried. Into the dried mixture, 72.11 g (1 mol) of isobutyl aldehyde was added and stirred. Controlling the reaction temperature in the range of 25-35°C, 40 g (1 mol) of oily (60%) sodium hydride was carefully added. Then, the reaction mixture was stirred over night at room temperature. After the reaction was ended, 500 g of water was added into the reaction mixture, the oil layer was then separated and the water layer was extracted twice with 300 ml of hexane. The organic layers were combined and washed with 300 ml of water, solvent was removed under reduced pressure, and then distilled under 2mm Hg to obtain 122.8 g of ethyl 8-methyl-6-nonenoate (Yield: 62% based upon the starting material).

The product was gradually added dropwise into 1 l of dried ether solution including 11.4 g (0.3 mol) of aluminum lithium hydride under nitrogen atmosphere. The dropwise addition was conducted while the reaction vessel was cooled sufficiently by ice water. After the addition was completed, the reaction temperature was returned to room temperature and stirred for 2 hours. After the reaction ended, 50 g of ethyl acetate was added to the reaction mixture and stirred for 10 minutes. Into the reaction mixture 300 ml of water was added, and the organic layer was separated. The water layer was adjusted to pH 7 and extracted three times with 200 ml of diethyl ether. The organic layers were combined and washed with 200 ml of water, and solvent was removed under reduced pressure to obtain 93.8 g (Yield 97%) of 8-methyl-6-nonen-1-ol.
Boiling Point: 82°C/3mm Hg
IR (liquid film, cm⁻¹): 3344, 2932, 1656, 1462, 1378, 1362
¹H-NMR (CDCl₃ solvent, TMS internal standard, δ):
0.94 (doublet, 6H, J=6.6Hz)
1.25-1.75 (multiplet, 7H)
1.92-2.15 (multiplet, 2H)
2.40-2.70 (multiplet, 1H)
3.63 (triplet, 2H, J=6.2Hz)
5.04-5.34 (multiplet, 2H)
MS(m/e, relative intensity): 69(100), 57(93), 67(83), 82(78),
81(77), 95(72), 41(65), 56(47), 68(34), 57(33)

### Example 2

### Preparation of 8-methyl nonanol [in formula (I), A= -CH₂CH₂-, B= -CH₂OH, a=b=0, c=4]:

93.8 g of 8-methyl-6-nonen-1-ol, which was prepared in example 1, and 14.1 g of 5% Palladium carbon were agitated for 1 hour in an autoclave at a reaction temperature of 60°C under 100 kg/cm² of hydrogen pressure. After the reaction ended, Palladium carbon was filtered off to obtain 91.0 g of 8-methyl nonanol (Yield 95.8%).
Boiling Point: 87°C/2.5 mmHg
IR (liquid film, cm⁻¹): 3340, 2927, 1467, 1384, 1367
¹H-NMR (CDCl₃ solvent, TMS internal standard, δ):
0.85 (doublet, 6H, J=6.2 Hz)
1.15-1.70 (multiplet, 7 H)
3.62 (multiplet, 2H)
MS(m/e, relative intensity): 56(100), 69(76), 55(69), 57(56),
43(36), 84(28), 97(23), 68(14)

### Example 3

### Preparation of 8-methyl nonanal [in formula (I), A= -CH₂CH₂-, B= -CHO, a=b=0, c=4]:

91.0g of 8-methyl nonanol, which was prepared in Example 2, was added dropwise into a mixture solution of 1.5 l of dichloromethane and 194 g (0.9 mol) of pyridinium chlorochromate, and the mixture was stirred for 2 hours at room temperature. Into the reaction mixture 3 l of diethyl ether was added, the mixture was passed through 500 g of florisil and solvent was evaporated off. The crude product obtained was distilled to obtain 77.5 g of 8-methyl nonanol (Yield 86.3%). Boiling point: 66°C/2 mmHg.
IR (liquid film, CM⁻¹): 2950, 1730, 1470, 1385, 1375
¹H-NMR (CD Cl₃ solvent, TMS internal standard, δ):
0.87 (doublet, 6H, J=6.2Hz)
1.20-1.72 (multiplet, 11H)
2.43 (triplet, 2H, J=7.5Hz)
9.76 (triplet, 1H, J-1.8Hz)
MS (m/e, relative intensity): 57(100), 82(65), 43(57), 69(55),
55(52), 56(49), 41(48), 72(42), 81(38), 95(37)

### Reference Example 1

### Preparation of 7-methyl-5-decen-1-ol [in formula (I), A= -CH=CH- B= -CH₂OH, a=2, b=0, c=3]:

104.54 g (0.5 mol) of ethyl 5-bromovalerate and 137.5 g (0.525 mol) of triphenyl phosphine were refluxed for 36 hours in 1 l of acetonitrile. After the reaction terminated, 500 ml of acetonitrile was distilled off, then the reaction mixture was dried. Under nitrogen atmosphere, 50.1 g (0.5 mol) of 2-methyl valeraldehyde was added, then the reaction mixture was stirred. Controlling the reaction temperature to within the range of 25-35°C, 20.0 g (0.5 mol) of oily (60%) sodium hydride was added little by little. After the addition, the reaction mixture was stirred over night at room temperature. After the reaction ended, 250 g of water was added into the reaction mixture, then oil layer was separated and the water layer was extracted three times with 150 ml of hexane. The organic layers were combined and washed with 150 ml of water. After the hexane solution was dried sufficiently with anhydrous Magnesium sulfate anhydrate, solvent was removed under reduced pressure, and distilled to obtain 57.3 g of ethyl 7-methyl-5-decenoate (Yield: 54%, based upon starting material). The product was gradually added dropwise into 500 ml of dried ethylether solution including 5.12 g (0.135 mol) of aluminum lithium hydride under nitrogen stream. The dropwise addition was done while a reaction vessel was cooled sufficiently by ice water. After the addition was completed, the reaction temperature was returned to room temperature and stirred for 2 hours. Then, 300 ml of water was added gradually into the reaction mixture, and the organic layer was separated. The water layer was returned to pH7 and extracted three times with 100 ml of diethylether. The organic layers were combined and washed with 100 ml of water, and solvent was removed from the reaction mixture under reduced pressure to obtain 44.2g of 7-methyl-5-decen-1-ol (Yield: 52'', based upon starting material).
IR (liquid film, cm⁻¹): 3345, 2958, 1457, 1066
¹H-NMR (CDCl₃ solvent, TMS internal standard, δ):
0.88 (triplet, 3H)
0.92 (doublet, 3H, J=6.6Hz)
1.15-1.35 (multiplet, 4H)
1.40-1.68 (multiplet, 5H)
1.96-2.15 (multiplet, 2H)
2.20-2.58 (multiplet, 1H)
3.36-3.70 (multiplet, 2H)
5.00-5.45 (multiplet, 2H)
MS (m/e, relative intensity): 67(100), 109(93), 55(90), 81(76),
95(42), 68(31), 41(30), 56(22), 82(20), 84(20)

### Reference Example 2

### Preparation of 7-methyl decanol [in formula (I), A= -CH₂CH₂-, B= -CH₂OH, a=2, b=0, c=3]:

44g (0.26 mol) of 7-methyl-5-decen-1-ol, which was prepared in Reference Example 1 and 6.6 g of 5% Palladium carbon were agitated for 1 hour in an autoclave at a reaction temperature of 60°C under 100 kg/cm² of hydrogen pressure. After the reaction ended, Palladium carbon was filtered off to obtain 7-methyl decanol.
Amount: 43.5 g (Yield 97%)
Boiling point: 93°C/3 mmHg)
IR (liquid film, cm⁻¹): 3340, 2926, 1463, 1378, 1058
¹H-NMR (CDCl₃ solvent, TMS internal standard, δ):
0.80-0.95 (multiplet, 6H)
1.18-1.70 (multiplet, 16H)
3.67 (triplet, 2H, J=6.3Hz)
MS (m/e, relative intensity): 69(100, 55(71), 84(49), 43(48),
70(43), 111(35), 56(33), 41(32), 83(29), 71(23)

### Reference Example 3

### Preparation of 7-methyl decanal [in the formula (I), A= -CH₂CH2-, B= -CHO, a=2, b=0, c=3]:

43.5 g (0.25 mol) of 7-methyl decanol, which was prepared in Example 5, was added into a mixture solution of 500 ml of dichloromethane and 81.9 g (0.38 mol) of pyridinium chlorochromate, and the reaction mixture was stirred for 2 hours at room temperature. Into the reaction mixture 2 l of diethyl ether was added, the mixture was passed through 300 g of florisil, and then solvent was evaporated off. The crude product obtained was distilled to obtain 7-methyl decanal.
Amount: 31.1 g (Yield: 73%):
Boiling point: 83.5°C/4 mmHg)
IR (liquid film, cm⁻¹): 2932, 1729, 1466, 1379
¹H-NMR (CDCl₃ solvent, TMS internal standard, δ):
0.8-0.92 (multiplet, 6H)
1.20-1.80 (multiplet, 13H)
2.43 (triplet, 2H, J=6.6Hz)
9.76 (triplet, 1H, J=1.8Hz)
MS (m/e, relative intensity): 109(100), 43(92), 55(88), 57(73),
84(71), 67(67), 41(58), 71(52), 85(50), 83(39)

### Example 4

### Preparation of 9-methyl-6-decen-1-ol [in formula (I), A= -CH=CH-, B= -CH₂OH, a=0, b=1, c=4]:

9-methyl-6-decen-1-ol was prepared in the same manner according to Reference Example 1 except that 111.56 g (0.5 mol) of ethyl 6-bromohexanoate was used in place of ethyl 5-bromovalerate and 43.07 g (0.5 mol) of isovaleraldehyde was used in place of 2-methyl valeraldehyde.
Amount: 42.6 g (Yield: 50%, based upon starting material)
IR (liquid film, cm⁻¹): 3336, 2932, 1465, 1383, 1363, 1055
¹H-NMR (CDCl₃ solvent, TMS internal standard, δ):
0.89 (doublet, 6H, J=6.2Hz)
1.24-1.64 (multiplet, 9H)
1.80-2.05 (multiplet, 3H)
3.62 (triplet, 2H)
5.32-5.45 (multiplet, 2H)
MS (m/e, relative intensity): 67(100), 55(65), 81(56), 95(48),
82(46), 69(41), 68(38), 41(37), 56(33), 43(30)

### Example 5

### Preparation of 9-methyl decanol [in formula (I), A= -CH₂CH₃-, B= -CH₂OH, a=0, b=1, c=4] and 9-methyl decanal [in formula (I), A= -CH₂CH₂-, B= -CH0, a= 0, b= 1, c= 4]:

42.6 g (0.25 mol) of 9-methyl-7-decen-1-ol, which was prepared in Example 5, was hydrogenated according to Reference Example 2 to obtain 9-methyl decanol (amount: 41.8 g, yield: 97%). The alcohol was oxidized according to Reference Example 3 to obtain 9-methyl decanal.
Amount: 34.3 g (Yield: 83%)
Boiling point: 77°C/3 mmHg
IR (liquid film, cm⁻¹): 2927, 1730, 1467, 1386, 1367
¹H-NMR (CDCl₃ solvent, TMS inner standard, δ):
0.86 (doublet, 6H, J=6.2Hz)
1.20-1.70 (multiplet, 13H)
2.43 (triplet, 1H, J=6.4Hz)
9.76 (triplet, 1H, J=2.0Hz)
MS (m/e, relative strength): 57(100), 43(74), 69(69)

### Test 1

The fragrances of the methyl branched aliphatic compounds (I), which are combined into the perfume composition of the present invention, were examined. The results are shown in Table 1.

**Table 1**

| Compound of the formula (I) | odor description |
|---|---|
| 8-methyl nonanal | citrus, sweet, waxy, milky |
| 9-methyl decanal | green, fatty, aldehyde |
| 8-methyl nonanol | green, fatty, floral, rosy |
| 9-methyl decanol | fatty, oily |

From Table 1, the fragrance of the aldehydes having a branched methyl group according to the present invention has, in comparison with the aldehydes without a branched methyl group, a characteristic smell which is reduced with respect to the particular aldehyde odor and enhanced with respect to the particular citrus and floral odors. The fragrances of the methyl branched alcohols also have strong floral, fruity and green leafy odors, unlike the usual alcohols.

### Test 2

In order to evaluate the strength of the fragrance, the odor threshold of the fragrance was measured by ten professional panelists.

### 〈test method〉

The odor threshold was evaluated by using the method of triangle comparison (answer one).

### 〈test result〉

The result regarding 8-methyl nonanal is described below. 2-methyl decanal and n-undecanol were used as comparative samples. Though the odor threshold of 2-methyl decanal and n-undecanol were both 5.0 ppm, the odor threshold of 8-methyl nonanal of the present invention was 0.01 ppm.

### Example 6

| ROSE BASE: | (parts by weight) |
|---|---|
| geranium bourbon | 10 |
| citronellol | 100 |
| geraniol | 50 |
| nerol | 30 |
| phenylethyl alcohol | 600 |
| diphenyl oxide | 5 |
| linalool | 15 |
| geranyl acetate | 10 |
| eugenol | 10 |
| decenol | 1 |
| phenylacetic acid 9.9% (phenylethyl alcohol) | 10 |
| methyl phenylacetate | 20 |
| bees wax absolute | 5 |
| dipropylene glycol | 124 |
| TOTAL | 990 |

Into 990 parts by total weight of rose base described above, 10 parts by weight of 8-methyl-6-nonen-1-ol was added, and ten professional panelists evaluated the added composition of the present invention and non-added comparative composition. All ten panelists found that the composition of the present invention has a stronger and enhanced natural floral rose base odor.

### Example 7

| MANDARIN BASE: | parts by weight |
|---|---|
| α-pinene | 20 |
| β-pinene | 10 |
| myrcene | 10 |
| limonene | 484 |
| lemon terpene | 100 |
| orange valencia | 200 |
| γ-terpinene | 150 |
| mandarin aldehyde 10(%)* | 10 |
| thyme red | 5 |
| dimethyl anthranilate | 5 |
| coumarin | 1 |
| carrot seed oil | 1 |
| geranyl methyl carbinyl acetate | 2 |
| TOTAL | 998 |

| | |
|---|---|
| * produced by Firmenich SA specialty chemical cis -3-dodecenal | |

Into 998 parts by total weight of Mandarin base described above, w parts by weight of 8-methyl nonanal was added, and ten professional panelists evaluated the added composition (the present invention) and non-added comparative composition. Nine panelists found that the composition of the present invention has much peel-like feeling and enhanced natural citrus odors.

### [Effect of the Invention]

Methyl branched aliphatic compounds (I) of the present invention have citrus, floral, green or fruity fragrances, and the fragrances are extremely strong. Therefore, the perfume composition of the present invention comprising a methyl branched aliphatic compound (I) can emit, for example, much peel-like odor to reach a more natural citrus-like perfume composition, and emit a sweet odor to reach a more natural floral-like perfume composition; and furthermore, emit a more transparent feeling as to a fruity perfume composition. Accordingly, the perfume composition of the present invention is superior as a material for a variety of combinations, and widely applicable to combination perfumes for dishwasher detergents, heavy duty detergents, softeners, fragrant agents, shampoos, rinses, cosmetics, soaps, etc.

## Claims

1. A combination perfume composition which comprises
a perfume base; and
a fragrance emitting effective amount of a compound selected from the group consisting of 8-methyl nonanal, 8-methyl decanal, 9-methyl decanal, 8-methyl nonanol, 8-methyl decanol, 9-methyl decanol, 8-methyl-6-nonen-1-ol and 9-methyl-6-decen-1-ol.

2. The composition according to claim 1 wherein the amount of said compound is in the range of 10 ppm to 5 wt% based on the total weight of the combination perfume.

3. A method for imparting a fragrance to a perfume base composition which comprises adding to said perfume base composition a fragrance imparting effective amount of a methyl branched aliphatic compound selected from the group consisting of 8-methyl nonanal, 8-methyl decanal, 9-methyl decanal, 8-methyl nonanol, 8-methyl decanol, 9-methyl decanol, 8-methyl-6-nonen-1-ol and 9-methyl-6-decen-1-ol.

## Patentansprüche

1. Zusammengesetzte Duftstoffzusammensetzung, umfassend einen Parfumgrundstoff, und
eine duftemittierende wirksame Menge einer Verbindung, ausgewählt aus der Gruppe, bestehend aus 8-Methylnonanal, 8-Methyldecanal, 9-Methyldecanal, 8-Methylnonanol, 8-Methyldecanol, 9-Methyldecanol, 8-Methyl-6-nonen-1-ol und 9-Methyl-6-decen-1-ol.

2. Zusammensetzung nach Anspruch 1, worin die Menge der Verbindung im Bereich von 10 ppm bis 5 Gew.-%, bezogen auf das Gesamtgewicht des zusammengesetzten Duftstoffs, beträgt.

3. Verfahren zur Verleihung eines Dufts an eine Parfumgrundstoffzusammensetzung, dadurch **gekennzeichnet**, daß man der Parfumgrundstoffzusammensetzung eine duftverleihende wirksame Menge einer methylverzweigten aliphatischen Verbindung, ausgewählt aus der Gruppe, bestehend aus 8-Methylnonanal, 8-Methyldecanal, 9-Methyldecanal, 8-Methylnonanol, 8-Methyldecanol, 9-Methyldecanol, 8-Methyl-6-nonen-1-ol und 9-Methyl-6-decen-1-ol, zusetzt.

## Revendications

1. Composition de parfum formée par combinaison qui comprend :
une base de parfum; et
une quantité efficace émettant une odeur agréable d'un composé choisi dans le groupe consistant en 8-méthyl nonanal, 8-méthyl décanal, 9-méthyl décanal, 8-méthyl nonanol, 8-méthyl décanol, 9-méthyl décanol, 8-méthyl-6-nonèn-1-ol et 9-méthyl-6-décèn-1-ol.

2. Composition suivant la revendication 1, dans laquelle la quantité de ce composé est dans la gamme de 10 ppm à 5% en poids par rapport au poids total du parfum formé par combinaison.

3. Procédé pour conférer une odeur agréable à une composition de base de parfum, qui comprend l'addition à cette composition de base de parfum d'une quantité efficace conférant une odeur agréable d'un composé aliphatique à ramification méthyle, choisi dans le groupe consistant en 8-méthyl nonanal, 8-méthyl décanal, 9-méthyl décanal, 8-méthyl nonanol, 8-méthyl décanol, 9-méthyl décanol, 8-méthyl-6-nonèn-1-ol et 9-méthyl-6-décèn-1-ol.
